(19) **Europäisches Patentamt**
**European Patent Office**
**Office européen des brevets**

(11) **EP 4 461 815 A1**

(12) **EUROPEAN PATENT APPLICATION**
published in accordance with Art. 153(4) EPC

(43) Date of publication:
**13.11.2024 Bulletin 2024/46**

(21) Application number: **22925247.3**

(22) Date of filing: **30.11.2022**

(51) International Patent Classification (IPC):
**C12Q 1/02** (2006.01)  **C12M 3/00** (2006.01)

(52) Cooperative Patent Classification (CPC):
**C12M 3/00; C12Q 1/02**

(86) International application number:
**PCT/JP2022/044238**

(87) International publication number:
**WO 2023/153058 (17.08.2023 Gazette 2023/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.02.2022 JP 2022020642**

(71) Applicant: **FUJIFILM Corporation**
**Tokyo 106-8620 (JP)**

(72) Inventors:
• **KASAI, Yusuke**
 **Ashigarakami-gun, Kanagawa 258-8577 (JP)**
• **OBA, Takahiro**
 **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **METHOD FOR EVALUATING CELLS AND EVALUATION DEVICE**

(57)    A cell evaluation method according to the disclosed technology includes culturing cells such that a plurality of cells or a plurality of cell aggregates are arranged on a culture surface, installing a flow channel attachable to and detachable from the culture surface, and allowing a liquid to flow through the flow channel to provide a liquid flow to each of the plurality of cells or the plurality of cell aggregates.

## FIG. 4B

EP 4 461 815 A1

**Description**

**BACKGROUND OF THE INVENTION**

1. Field of the Invention

The disclosed technology relates to a cell evaluation method and an evaluation device.

2. Description of the Related Art

The following technologies are known as technologies relating to a cell evaluation method. For example, JP2012-100642A describes an evaluation method for cultured cells in which, in cells cultured on a surface of a base material, an adhesion force between the surface of the base material and cells is measured using a shear stress of a fluid.
WO2015/125742A describes a cell culture container having a meandering passage through which a liquid passes, in which, on a bottom surface of the passage, a plurality of cell seeding regions in which cells passing through the passage are seeded are provided at equal intervals along the passage.
T. Ohashi et al., JSME Inter. J. Series C, 43(4), 780-786, 2000 describes that endothelial cells are cultured in a cell culture dish, and a single layer of the endothelial cells is loaded into a parallel plate flow chamber having a flow section of a width of 14 mm and a height of 0.5 mm, thereby being exposed to a shear stress.

**SUMMARY OF THE INVENTION**

It is known that adhesive cells exhibit various responses such as alignment, differentiation, peeling, or cell death with respect to a flow field, and the adhesion characteristics of cells are important evaluation indicators in the examination of culture conditions and quality evaluation of cells. In addition, the evaluation of the adhesiveness of cells is also required in the research and development of a culture substrate or a cell adhesive and is a matter of great concern in terms of both the use application in research and the use application in industry. Therefore, in a wide range of fields related to adhesive cells, there is a demand for a technology for simply and efficiently evaluating the response of adhesive cells to a liquid flow.
As described in JP2012-100642A and WO2015/125742A, in a case where a closed micro flow channel is used, it is necessary to introduce cells into the closed micro flow channel; however, it is not easy to compartmentalize a plurality of cells or a plurality of cell aggregates in the inside of the closed micro flow channel and arrange them along the flow channel. In addition, there is a concern that cells may be damaged in a case where the cells are introduced into the micro flow channel. Further, there is a concern that in the closed micro flow channel, culture medium in a culture medium may be non-uniform, and thus the environment may be different from a standard culture environment. As a result, there is a concern that the state of the cells may be affected, and thus the evaluation result may be affected. In addition, in the technology according to T. Ohashi et al., JSME Inter. J. Series C, 43(4), 780-786, 2000, a single endothelial cell single layer is targeted for treatment, and thus a plurality of targets are not collectively treated, which makes it difficult to carry out an efficient cell evaluation.
The disclosed technology has been made in consideration of the above points, and an object of the disclosed technology is to simply and efficiently evaluate the response of adhesive cells to a liquid flow.
A cell evaluation method according to the disclosed technology includes culturing cells such that a plurality of cells or a plurality of cell aggregates are arranged on a culture surface, installing a flow channel attachable to and detachable from the culture surface, and allowing a liquid to flow through the flow channel to provide a liquid flow to each of the plurality of cells or the plurality of cell aggregates.
The flow channel may be formed by closely attaching a surface of a flow channel member having a groove constituting the flow channel on the surface, to the culture surface.
The flow channel member may be subjected to registration such that the plurality of cells or the plurality of cell aggregates are arranged along a flow direction of the liquid. The registration of the flow channel member may be carried out using a first alignment mark provided on a surface of the flow channel member and a second alignment mark provided on the culture surface.
A shear stress applied to each of the plurality of cells or the plurality of cell aggregates due to the liquid flow may change along a flow direction of the liquid. A shear stress applied to each of the plurality of cells or the plurality of cell aggregates due to the liquid flow may change linearly along a flow direction of the liquid. A shear stress applied to each of the plurality of cells or the plurality of cell aggregates due to the liquid flow may be constant at each position in the flow direction of the liquid.
An area of a cross section of the flow channel intersecting with the flow direction of the liquid may change along the flow direction of the liquid. An area of a cross section of the flow channel intersecting with the flow direction of the liquid may expand from an upstream side toward a downstream side in the flow direction of the liquid.

The cell evaluation method according to the disclosed technology may include carrying out cell culture such that the plurality of cells or the plurality of cell aggregates form a predetermined pattern on the culture surface. The cell evaluation method according to the disclosed technology may include attaching a sheet having a plurality of opening portions to a culture surface and forming a plurality of cells or a plurality of cell aggregates a portion of the culture surface, which is exposed in the opening portion. The plurality of cells or the plurality of cell aggregates may have the same size.

The evaluation device according to the disclosed technology is an evaluation device for providing a liquid flow to a plurality of cells or a plurality of cell aggregates, which are arranged on a culture surface, where a flow channel attachable to and detachable from the culture surface is formed, and a liquid is allowed to flow through the flow channel to provide a liquid flow to each of the plurality of cells or the plurality of cell aggregates.

An evaluation device according to the disclosed technology may include a flow channel member having, on a surface, a groove constituting the flow channel. The surface of the flow channel member which has the groove is closely attached to the culture surface to form the flow channel.

The flow channel may be configured such that a shear stress applied to each of the plurality of cells or the plurality of cell aggregates changes along a flow direction of the liquid. The flow channel may be configured such that a shear stress applied to each of the plurality of cells or the plurality of cell aggregates changes linearly along a flow direction of the liquid.

An area of a cross section of the flow channel intersecting with the flow direction of the liquid may change along the flow direction of the liquid. An area of a cross section of the flow channel intersecting with the flow direction of the liquid may expand from an upstream side toward a downstream side in the flow direction of the liquid.

The evaluation device according to the embodiment of the disclosed technology may further include an attachment unit to which the flow channel member is attached, a holding part that holds the attachment unit to be rotatable or movable, and a fixing unit that has a mounting region, in which a substrate having the culture surface is mounted, and fixes the holding part to a position corresponding to the mounting region.

The flow channel member and the attachment unit may be formed from a material having a light-transmitting property.

According to the disclosed technology, it is possible to simply and efficiently evaluate the response of adhesive cells to a liquid flow since a liquid flow is provided to a plurality of cells or a plurality of cell aggregates arranged on any substrate, by using a flow channel attachable to and detachable from the culture surface.

## BRIEF DESCRIPTION OF THE DRAWINGS

Fig. 1 is a perspective view showing one example of a configuration of a masking sheet that is used for patterned culture of cells according to an embodiment of the disclosed technology.

Fig. 2A is a view showing one example of a cell culture method according to an embodiment of the disclosed technology.

Fig. 2B is a view showing one example of the cell culture method according to the embodiment of the disclosed technology.

Fig. 2C is a view showing one example of the cell culture method according to the embodiment of the disclosed technology.

Fig. 2D is a view showing one example of the cell culture method according to the embodiment of the disclosed technology.

Fig. 2E is a view showing one example of the cell culture method according to the embodiment of the disclosed technology.

Fig. 2F is a view showing one example of the cell culture method according to the embodiment of the disclosed technology.

Fig. 2G is a view showing one example of the cell culture method according to the embodiment of the disclosed technology.

Fig. 3A is a perspective view showing one example of a configuration of a flow channel member according to an embodiment of the disclosed technology.

Fig. 3B is a plan view showing one example of the configuration of the flow channel member according to an embodiment of the disclosed technology.

Fig. 4A is a view showing a state in which the flow channel member is installed on a culture surface.

Fig. 4B is a view showing a state in which the flow channel member is installed on the culture surface.

Fig. 5 is a perspective view showing one example of a configuration of a first alignment mark and a second alignment mark according to embodiments of the disclosed technology.

Fig. 6 is a plan view showing a state in which a shear stress due to a liquid flow is applied to cell aggregates.

Fig. 7 is a graph showing one example of a relationship between a position of a liquid in a flow direction, the liquid flowing through the flow channel according to the embodiment of the disclosed technology, and a shear stress generated due to a liquid flow.

Fig. 8 is a view in which a cross section of the flow channel according to the embodiment of the disclosed technology is shown on a coordinate plane.

Fig. 9 is a view showing one example of a situation in which cell aggregates are peeled off by a shear stress.

Fig. 10 is a view showing one example of a relationship between a configuration of the flow channel according to the embodiment of the disclosed technology, a position of a liquid in a flow direction, where the liquid flows through the flow channel, and a shear stress generated due to the liquid flow.

Fig. 11 is a view showing one example of the relationship between a configuration of the flow channel according to the embodiment of the disclosed technology, a position of a liquid in a flow direction, where the liquid flows through the flow channel, and a shear stress generated due to the liquid flow.

Fig. 12A is a cross-sectional view showing one example of a configuration of an evaluation device according to an embodiment of the disclosed technology.

Fig. 12B is a cross-sectional view showing one example of the configuration of the evaluation device according to the embodiment of the disclosed technology.

Fig. 13A is a perspective view showing one example of a configuration of an attachment unit according to an embodiment of the disclosed technology.

Fig. 13B is a plan view showing one example of the configuration of the attachment unit according to the embodiment of the disclosed technology.

Fig. 13C is a cross-sectional view taken along a line 13C-13C in Fig. 13B.

Fig. 13D is a cross-sectional view showing a state in which a flow channel member is attached to the attachment unit according to the embodiment of the disclosed technology.

Fig. 14A is a perspective view showing one example of a configuration of a holding part in a state in which the attachment unit according to the embodiment of the disclosed technology is held.

Fig. 14B is a plan view showing one example of the configuration of the holding part in a state in which the attachment unit according to the embodiment of the disclosed technology is held.

Fig. 14C is a cross-sectional view taken along a line 14C-14C in Fig. 14B.

Fig. 15A is a perspective view showing one example of a configuration of a base unit constituting a holding part according to an embodiment of the disclosed technology.

Fig. 15B is a perspective view showing one example of a configuration of a lid portion constituting the holding part according to the embodiment of the disclosed technology.

Fig. 16 is a plan view showing one example of the configuration of the flow channel member according to an embodiment of the disclosed technology.

Fig. 17 shows photomicrographic images of cell colonies patterned by patterned culture according to Example of the disclosed technology.

Fig. 18 is a view showing a flow channel member according to Example of the disclosed technology.

Fig. 19 is a view showing an attachment unit in a state of being held by a holding part according to Example of the disclosed technology.

Fig. 20 is a view showing an evaluation device according to Example of the disclosed technology.

Fig. 21 shows photomicrographic images showing a state in which cell colonies arranged on a culture surface according to Example of the disclosed technology are peeled off by a shear stress due to a liquid flow.

Fig. 22 shows photomicrographic images showing a state of the entire flow channel after providing a liquid flow according to Example of the disclosed technology.

**DESCRIPTION OF THE PREFERRED EMBODIMENTS**

Hereinafter, one example of the embodiment of the disclosed technology will be described with reference to the drawings. It is noted that the same or equivalent constitutional elements and portions in the drawings are assigned by the same reference numerals, and overlapping descriptions will be omitted.

A cell evaluation method according to an embodiment of the disclosed technology includes a first step of culturing cells on a culture surface such that a plurality of cells or a plurality of cell aggregates are arranged on a culture surface, a second step of installing a flow channel attachable to and detachable from the culture surface, and a third step of allowing a liquid to flow through the flow channel to provide a liquid flow to each of the plurality of cells or the plurality of cell aggregates. Hereinafter, details of the first to third steps described above will be described.

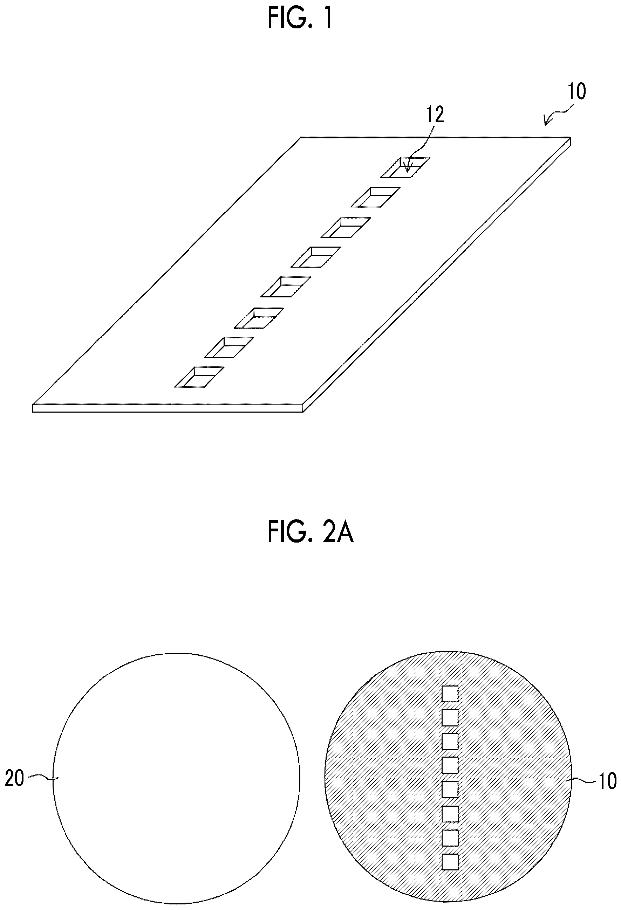

In the first step, a patterned culture technology using a masking sheet can be used in order to culture cells so that cells or cell aggregates are regularly arranged on a culture surface. Fig. 1 is a perspective view showing one example of a configuration of a masking sheet 10 that is used for patterned culture of cells.

The masking sheet 10 is formed from a material having adhesiveness with respect to a surface (culture surface) of a substrate (not shown in the drawing) that is used for the adhesion culture of cells. The masking sheet 10 preferably has sufficient flexibility in order to ensure the adhesiveness to the culture surface. As a material of the masking sheet 10, for example, a rubber member such as silicone rubber (VMQ, PVMQ, or FVMQ), isoprene rubber (IR), or fluororubber (FKM) can be suitably used. The masking sheet 10 includes a plurality of opening portions 12 that penetrate through a base material. The number, shape, and size of the opening portion 12, and the interval between the adjacent opening portions 12 can be freely determined. Cells or cell aggregates to be subjected to patterned culture are formed on the culture surface to have a pattern corresponding to the pattern of the opening portion 12 of the masking sheet 10. The masking sheet 10 may have a plurality of the opening portions 12 that have the same shape and the same size and are arranged to be spaced by a gap. According to such a pattern of the opening portion 12, it is possible to compartmentalize cells or cell aggregates and carry out culture or evaluation for each compartmentalized unit of cells. In addition, it is possible to quantify or standardize the state of the cells in each compartment. Fig. 1 shows an example of a configuration in which a plurality of the rectangular opening portions 12 having the same size are arranged side by side on a straight line.

Fig. 2A to Fig. 2G are views showing one example of a cell culture method in which a patterned culture is carried out using the masking sheet 10. First, a substrate 20 and the masking sheet 10 are prepared (Fig. 2A). A surface of the substrate 20 constitutes a culture surface. A commercially available cell culture dish can be used as the substrate 20. The masking sheet 10 may be cut into the same shape as the shape of the substrate 20 (the circular shape in the example shown in Fig. 2A).

The diameter of the masking sheet 10 is preferably the same as the diameter of the substrate 20. As a result, the region in which a cell adhesive 30 adheres and the region in which a cell 40 adheres, other than the opening portion 12, are reduced.

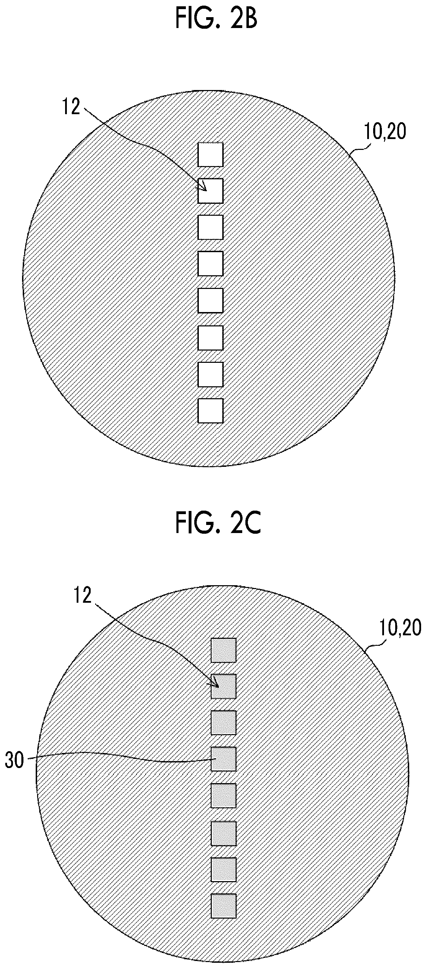

Next, the masking sheet 10 is attached to the surface (culture surface) of the substrate 20 (Fig. 2B). Specifically, the masking sheet 10 is brought into contact with the surface (culture surface) of the substrate 20, and then a load is applied to the masking sheet 10 to closely attach the masking sheet 10 to the substrate 20. The flexibility of the masking sheet 10 ensures high adhesiveness, and the masking sheet 10 is attached to the substrate 20 without generating air bubbles between the masking sheet 10 and the substrate 20. The surface (culture surface) of the substrate 20 is exposed in the opening portion 12 of the masking sheet 10.

Next, the cell adhesive 30 is applied onto the surface (culture surface) of the substrate 20 using the masking sheet 10 as a mask (Fig. 2C). As a result, the cell adhesive 30 adheres to a portion of the surface (culture surface) of the substrate 20, the portion being exposed in the opening portion 12 of the masking sheet 10. As a result, a pattern of the cell adhesive 30, which corresponds to the pattern of the opening portion 12 of the masking sheet 10, is formed on the culture surface. As the cell adhesive 30, it is possible to use an extracellular matrix protein such as vitronectin, laminin, fibronectin, collagens, or gelatins.

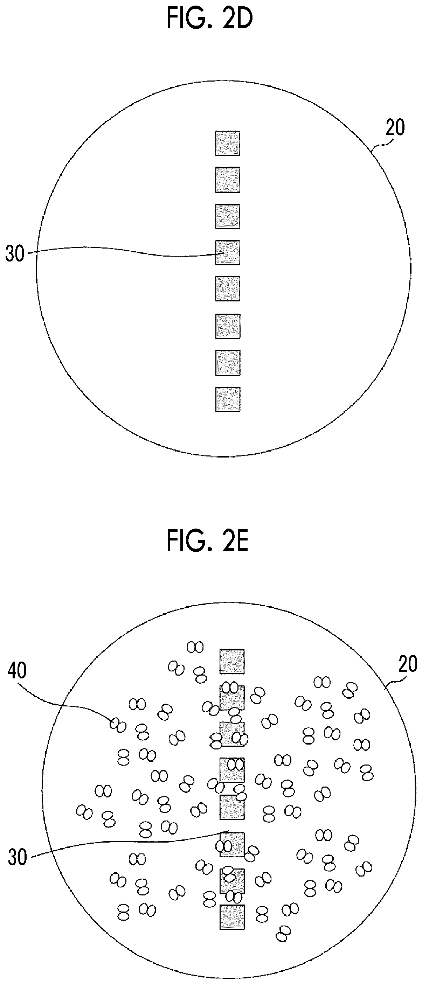

Next, the cell adhesive 30 is substituted with a phosphate buffer solution, and then the masking sheet 10 is peeled off from the culture surface (Fig. 2D). The cell adhesive 30 subjected to patterning is maintained on the surface (culture surface) of the substrate 20. Next, a blocking agent is applied onto the surface (culture surface) of the substrate 20. This treatment is a blocking treatment for inhibiting non-specific cell adhesion in a region other than the region in which the cell adhesive 30 has adhered to the culture surface. As the blocking agent, for example, a bovine serum albumin solution prepared at a predetermined concentration can be used.

Next, the cells 40 adjusted to a predetermined density are seeded on the surface (culture surface) of the substrate 20 (Fig. 2E). The cell 40 may be any adhesive cell, and it is possible to use, for example, stem cells such as induced pluripotent stem cells (iPS cell), an embryonic stem cell (ES cell), a mesenchymal stem cell, and a hematopoietic stem cell, somatic cells such as a lung cell, a stomach cell, a liver cell, a kidney cell, an intestinal cell, a nerve cell, an eye cell, a vascular endothelial cell, a myocardial cell, and a bone cell, cancer cells such as a lung cancer cell, a stomach cancer cell, a colon cancer cell, a breast cancer cell, a leiomyosarcoma cell, a fibrosarcoma cell, and an osteosarcoma cell, and established cell lines such as an HEK293 cell (human embryonic kidney cell) and a Vero cell (African green monkey kidney cell). Typically, an iPS cell derived from a human can be used. In addition, a cell derived from an animal (specifically, a mouse, a rat, a rabbit, a pig, a dog, a monkey, a cow, a horse, a sheep, a chicken, and the like) other than a human may be used.

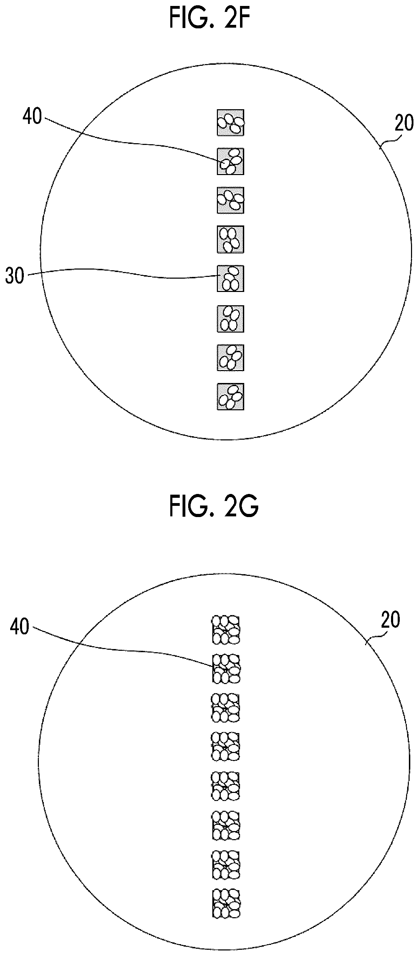

Thereafter, the static culture of the cell 40 is carried out. During the culture period, the substrate 20 on which the cells 40 are seeded is accommodated in an incubator that is maintained at an appropriate temperature and an appropriate $CO_2$ concentration. The cell 40 adheres in a region of the surface (culture surface) of the substrate 20, in which the cell adhesive 30 has adhered (Fig. 2F).

The culture is continued while carrying out the medium replacement at a predetermined timing. As a result, the cell 40 proliferates in a region of the surface (culture surface) of the substrate 20, in which the cell adhesive 30 has adhered. As a result, it is possible to obtain cells or cell aggregates (colonies), which have a pattern corresponding to the pattern of the opening portion 12 of the masking sheet 10 (Fig. 2G). It is noted that in the present embodiment, although a case where the masking sheet 10 is peeled off from the culture surface after applying the cell adhesive 30 and before seeding the cell 40 has been shown as an example, the masking sheet 10 may be peeled off from the culture surface after applying the cell adhesive 30 and after seeding the cell 40.

In the second step, a flow channel, which is attachable to and detachable from the culture surface on which cells or cell aggregates are arranged by the above-described patterned culture, is installed.

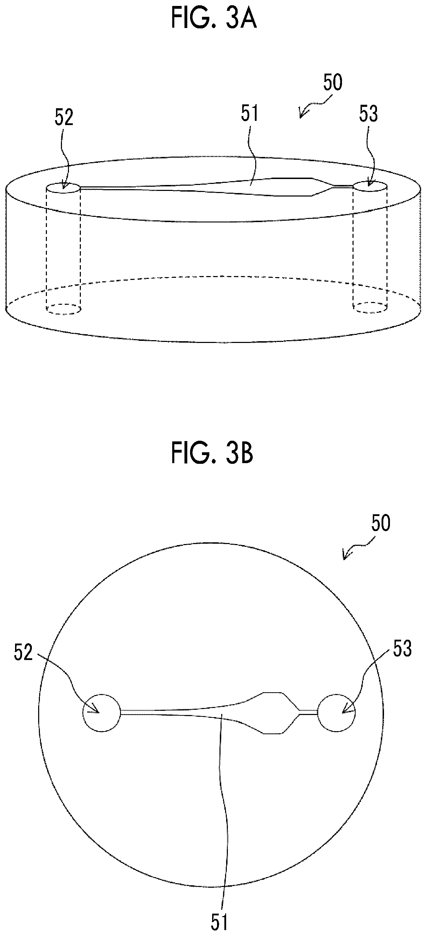

Figs. 3A and 3B are respectively a perspective view and a plan view, which show one example of a configuration of a flow channel member 50 for forming a flow channel attachable to and detachable from a culture surface.

The outer shape of the flow channel member 50 is not particularly limited; however, it preferably has a shape corresponding to the shape of the culture surface. Fig. 3A shows an example of the cylindrical flow channel member 50, assuming that cell culture is carried out using a commercially available dish having a circular culture surface.

A groove 51 that constitutes the flow channel is provided on one surface of the flow channel member 50. An inflow port 52 is connected to one end of the groove 51, and an outflow port 53 is connected to the other end of the groove 51. The inflow port 52 and the outflow port 53 are drawn out to a surface of the flow channel member 50 opposite to the surface on which the groove 51 is formed. It is preferable that the flow channel member 50 is formed from a material that is easily processed and has high adhesiveness to the culture surface. In addition, it is preferable that the flow channel member 50 is formed from a material having a light-transmitting property. As a material of the flow channel member 50, polydimethylsiloxane (PDMS) can be suitably used. The flow channel member 50 can be formed by, for example, cast molding.

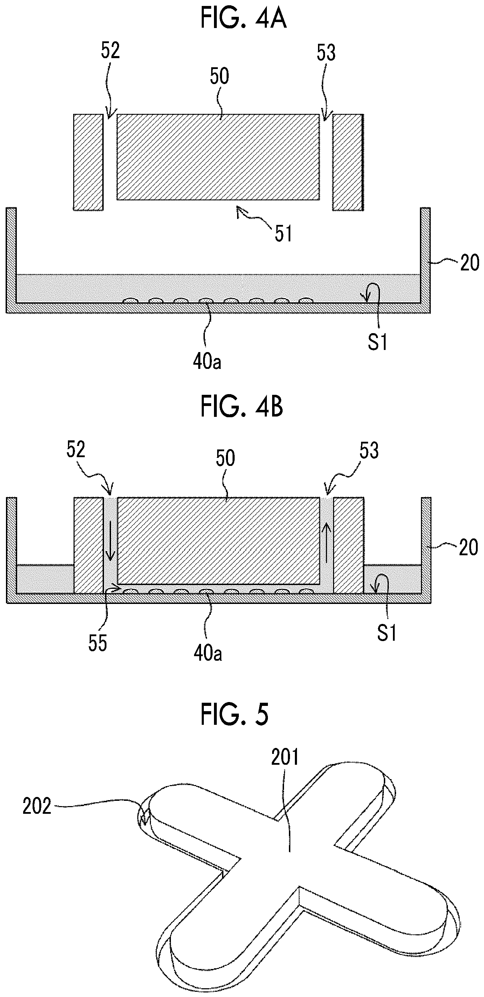

Figs. 4A and 4B are each a cross-sectional view showing a state in which the flow channel member 50 is installed on a culture surface S1 of the substrate 20 on which cell aggregates 40a are arranged. As shown in Fig. 4B, a flow channel 55 is formed on the culture surface S1 by closely attaching a surface of the flow channel member 50 on which the groove 51 is formed, to the culture surface S1. The flow channel 55 may be a micro flow channel having a height and a width, each of which has an order of magnitude of several hundred meters. It is noted that the flow channel member 50 can be easily detached from the culture surface S1, and the attachment to and detachment from the culture surface S1 can be repeated.

In a case where the flow channel member 50 is installed on the culture surface S1, the flow channel member 50 is subjected to registration such that the plurality of cell aggregates 40a formed on the culture surface S1 are arranged along the flow direction (that is, the flow channel direction) of the liquid that flows through the flow channel 55. Typically, each of the plurality of cell aggregates 40a are the registration is carried out such that positioned on the center line of the flow channel 55 in the width direction. In a case where the flow channel member 50 is formed from a material having a light-transmitting property, the positions of both the groove 51 and the cell aggregates 40a can be visually recognized through the flow channel member 50, which makes it easy to carry out the registration of the flow channel member 50.

The registration of the flow channel member 50 may be carried out using an alignment mark. Fig. 5 is a perspective view showing one example of a configuration of a first alignment mark 201 and a second alignment mark 202, which are used for the registration of the flow channel member 50. The first alignment mark 201 is provided on a surface of the flow channel member 50 on which the groove 51 is formed. The first alignment mark 201 has a convex pattern that protrudes from the surface of the flow channel member 50 on which the groove 51 is formed. The second alignment mark 202 is provided on the culture surface. The second alignment mark 202 has a concave pattern corresponding to the first alignment mark 201. The registration of the flow channel member 50 is carried out such that the first alignment mark 201 having a convex shape is fitted into the second alignment mark 202 having a concave shape. Although Fig. 5 shows an example of a cross-shaped pattern as the pattern of the first alignment mark 201 and the second alignment mark 202, any pattern can be used.

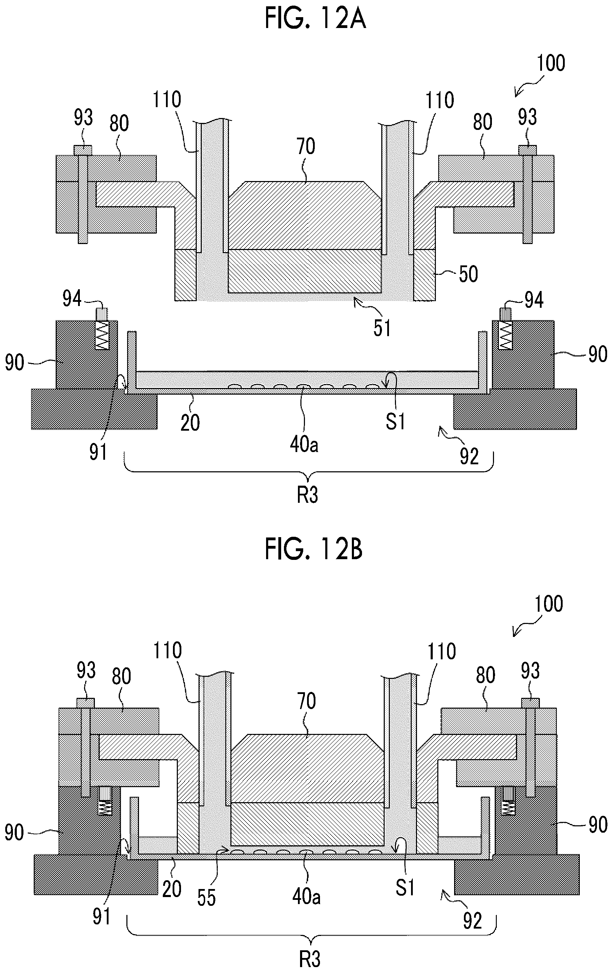

The second alignment mark 202 may be formed on the masking sheet 10. That is, the masking sheet 10 may have not only the opening portion 12 (see Fig. 1) for patterning cells or cell aggregates but also an opening portion that constitutes the second alignment mark 202. In this case, by providing a cutting line between the opening portion 12 of the masking sheet 10 and the alignment mark 202, and cutting along the cutting line, in the patterned culture, a portion of the masking sheet 10, on which the opening portion 12 is formed, a portion on which the second alignment mark 202 is formed may be left on the culture surface while peeling off the portion on which second alignment mark 202 is formed. In the masking sheet 10, the relative positional relationship between the opening portion 12 for patterning cells or cell aggregates and the second alignment mark 202 is fixed. Therefore, in a case where the registration of the flow channel member 50 is carried out using the second alignment mark 202, it is possible to match the disposition of the flow channel 55 to the cells or the cell aggregates, which are patterned on the culture surface. In addition, the registration of the flow channel member 50 can be easily carried out by using an evaluation device 100 (see Figs. 12A and 12B) described later.

It is noted that in the above description, although a case where the registration of the flow channel member 50 is carried out using a combination of the first alignment mark 201 having a convex shape and the second alignment mark 202 having a concave shape has been described as an example, the registration of the flow channel member 50 may be carried out using a combination of the first alignment mark having a concave shape and the second alignment mark having a concave shape. In addition, the registration of the flow channel member 50 may be carried out using both a combination of the first alignment mark 201 having a convex shape and the second alignment mark having a concave shape, and a combination of the first alignment mark having a concave shape and the second alignment mark having a concave shape. In this case, first, rough registration may be carried out using a combination of the first alignment mark having a convex shape and the second alignment mark having a concave shape, and then exact registration may be carried out using a combination of the first alignment mark having a concave shape and the second alignment mark having a concave shape.

In the third step, the liquid is introduced from the inflow port 52 of the flow channel member 50 using a means for feeding of liquid such as a pump to allow the liquid to flow through the flow channel 55, thereby providing a liquid flow to each of the plurality of cells or the plurality of cell aggregates, which are arranged on the culture surface S1. In the present embodiment, a case where the evaluation of the response of cell aggregates is carried out in a case where a shear stress due to a liquid flow is applied to each of a plurality of cell aggregates is shown as an example; however, the parameter that is allowed to act on the cells or cell aggregates due to the liquid flow is not limited to the shear stress. For example, the water pressure (that is, the pressure acting on the cell surface in the central direction of the cell), the pressure drag (that is, the force acting on the cell surface in the liquid flow direction perpendicular to the liquid flow direction), the culture medium concentration, the cell detachment agent concentration, or the like may be used as a parameter that is allowed to act on the cells or the cell aggregates.

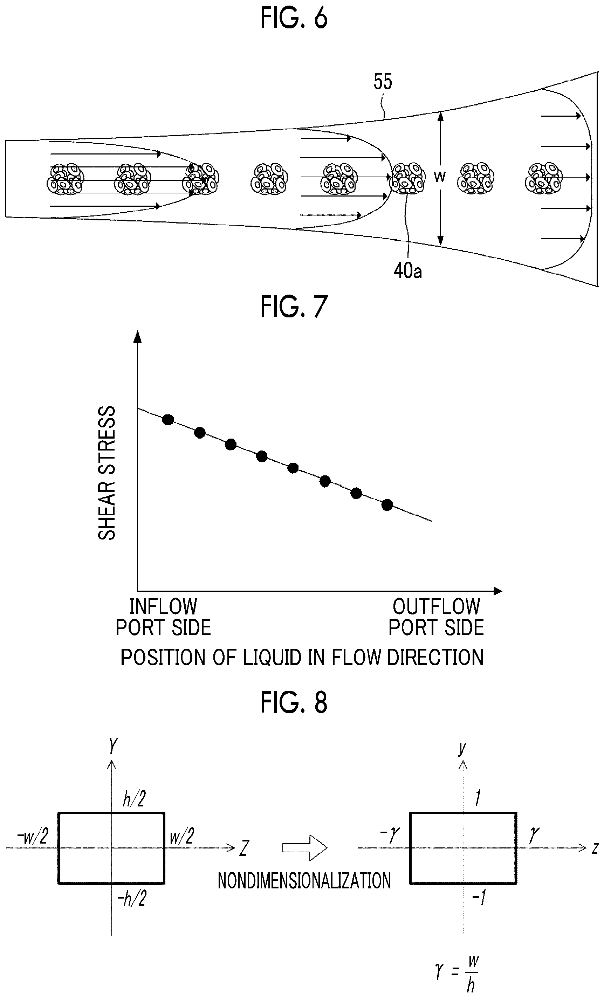

Fig. 6 is a plan view showing a state in which a liquid is allowed to flow through the flow channel 55 formed on the culture surface to apply a shear stress due to a liquid flow to each of the plurality of cell aggregates 40a which are arranged on the culture surface. The plurality of cell aggregates 40a are arranged along the liquid flow direction indicated by the arrow in Fig. 6.

Fig. 7 is a graph showing one example of a relationship between a position of a liquid in a flow direction, where the liquid flows through the flow channel 55, and a shear stress generated due to a liquid flow. A plurality of plots in Fig. 7 respectively correspond to the cell aggregates 40a arranged on the culture surface. In the present embodiment, as shown in Fig. 7, the shear stress applied to each of the plurality of cell aggregates 40a changes linearly along the flow direction of the liquid. More specifically, the shear stress is linearly reduced from the inflow port 52 side (upstream side) toward the outflow port 53 side (downstream side) of the flow channel 55. The change in the shear stress along the flow direction of the liquid is realized by changing, along the flow direction of the liquid, an area (hereinafter, referred to as a flow channel cross-sectional area) of a cross section (hereinafter, referred to as a flow channel cross section) of the flow channel 55, the cross section being intersecting the flow direction of the liquid along the flow direction of the liquid.

In the present embodiment, the shape of the flow channel cross section is rectangular, and the height of the flow channel 55 is constant over the entire region. The width of the flow channel 55 continuously increases from the upstream side to the downstream side in the liquid flow direction. That is, the flow channel cross-sectional area continuously increases from the upstream side to the downstream side in the liquid flow direction. In the present embodiment, as shown in Fig. 6, the outer shape line of the side surface of the flow channel 55 in planar view is set to be curved in order to linearly change the magnitude of the shear stress along the flow direction of the liquid.

A relationship between a shear stress $\sigma$ [Pa] generated due to the liquid flow and a width w [m] of the flow channel 55 is represented by Expression (1) in a case where the width of the flow channel is sufficiently large with respect to the height of the flow channel. In Expression (1), $\mu$ is a viscosity [Pa s] of the liquid that flows through the flow channel 55, and Q is a flow rate [m$^3$/sec] of the liquid that flows through the flow channel 55. x is a position [m] in the flow direction of the liquid that flows through the flow channel 55, and h is a height [m] of the flow channel 55. The cross-sectional shape of the flow channel 55 is rectangular.

$$\sigma\left(w(x)\right) = \frac{6\mu Q}{h^2 w(x)} \quad \cdot \; \cdot \; \cdot \quad (1)$$

By the way, Expression (1) can be applied in a case where the width w is sufficiently larger than the height h. In such a case, the flow rate Q required to obtain a desired shear stress is increased, and the amount of the liquid required is increased. In addition, the total area of the flow channel increases, and thus the area efficiency decreases. It is noted that the phrase "the width w is sufficiently larger than the height h" refers to that, for example, the width w is 10 times or more of the height h. In order to solve the above-described problem, the flow channel is designed by using an exact solution of the shear stress that can be applied even in a case where the ratio of the width w to the height h is small. The left of Fig. 8 is a view in which a cross section of the flow channel 55 having a rectangular cross section having a width w [m] and a height h [m] is shown on a coordinate plane, and the right of Fig. 8 is a view in which the cross section of the flow channel 55 is shown by nondimensionalization. In Fig. 8, $\Gamma = w/h$ is satisfied. The exact solution u(y,z) of the flow velocity distribution of the liquid that flows through the nondimensionalized flow channel 55 having a rectangular cross section is represented by Expression (2). In expression (2), m = (2n + 1)$\pi$/2 is satisfied.

$$u(y,z) = \frac{1 - y^2 - 4\sum_{n=0}^{\infty} \frac{(-1)^n \cos(my)\cosh(mz)}{m^3 \cosh(m\Gamma)}}{1 - 4\sum_{n=0}^{\infty} \frac{(-1)^n}{m^3 \cosh(m\Gamma)}} \qquad \cdot \cdot \cdot \quad (2)$$

Expression (3) is obtained by simplifying Expression (2). In Expression (3), $\alpha$ is represented by Expression (4), and $\beta(n)$ is represented by Expression (5).

$$u(y,z) = \frac{1}{\alpha}\left(1 - y^2 - 4\sum_{n=0}^{\infty} \beta \cos(my)\cosh(my)\right) \qquad \cdot \cdot \cdot \quad (3)$$

$$\alpha = 1 - 4\sum_{n=0}^{\infty} \beta \qquad \cdot \cdot \cdot \quad (4)$$

$$\beta(n) = \frac{(-1)^n}{m^3 \cosh(m\Gamma)} \qquad \cdot \cdot \cdot \quad (5)$$

The exact solution u(y,z) of the flow velocity distribution of the liquid that flows through the nondimensionalized flow channel 55 is dimensionalized as shown in Expressions (6) to (9).

$$U(Y,Z) = \frac{\overline{U}}{\overline{u}} u(Y,Z) \qquad \cdot \cdot \cdot \quad (6)$$

$$y = \frac{2Y}{h}, z = \frac{2Z}{h} \qquad \cdot \cdot \cdot \quad (7)$$

$$\overline{U} = \frac{Q}{wh} \qquad \cdot \cdot \cdot \quad (8)$$

$$\overline{u} = \frac{1}{\alpha\Gamma}\left(\frac{2}{3}\Gamma - 4\sum_{n=0}^{\infty} \frac{\beta}{m^2}\sin(m)\sinh(m\Gamma)\right) \qquad \cdot \cdot \cdot \quad (9)$$

The flow velocity distribution represented by Expression (6) to Expression (9) is converted into a shear stress $\tau$(w, h, Q) as in Expression (10).

$$\tau_{Y=h/2,Z=0} = \mu\frac{\partial U}{\partial Y}\bigg|_{Y=h/2,Z=0} = \frac{2\mu}{h}\frac{\overline{U}}{\overline{u}}\frac{1}{\alpha}\left(-2 + 4\sum_{n=0}^{\infty} \beta m\sin(m)\right) \qquad \cdot \cdot \cdot \quad (10)$$

The flow rate Q and the height h of the flow channel 55 are fixed to any value using Expression (10), and a relationship w(x) between the width w and the position x of the flow channel 55 is determined in terms of numerical analysis such that $\tau$ = ax + b is satisfied using a predetermined constants a and b, whereby as shown in Fig. 7, it is possible to construct such a flow channel 55 in which the shear stress due to the liquid flow changes linearly along the flow direction of the liquid.

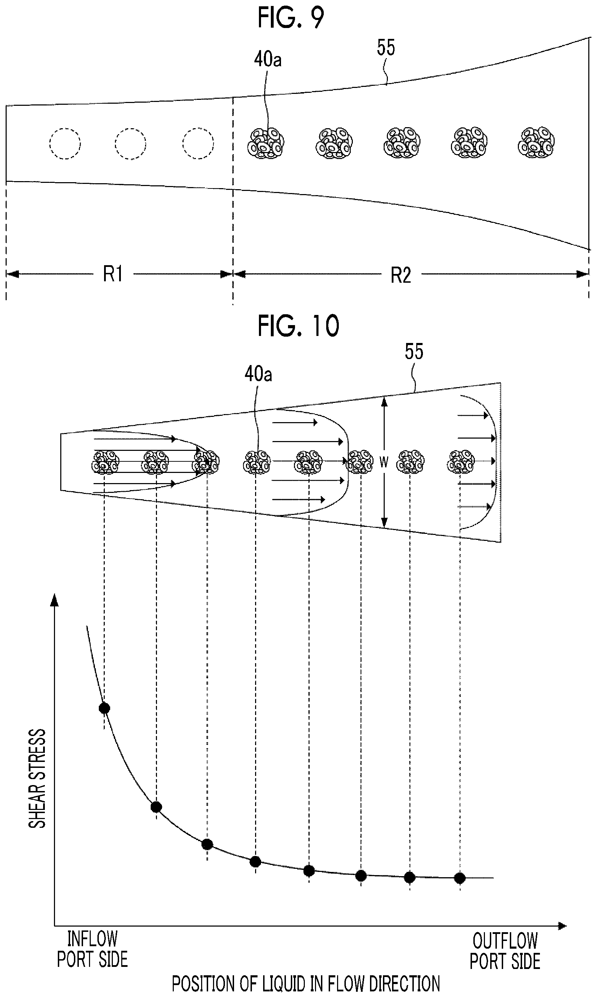

In a case where the shear stress due to the liquid flow exceeds the adhesive force of the cell aggregates 40a adhering to the culture surface, the cell aggregates 40a are peeled off from the culture surface. According to the evaluation method according to the present embodiment, it is assumed that, as shown in Fig. 9, some cell aggregates 40a positioned on the inflow port side (upstream side) of the flow channel 55 on which a relatively large shear stress acts are peeled off from the culture surface, and some cell aggregates 40a positioned on the outflow port side (downstream side) of the flow channel 55 on which a relatively small shear stress acts remain on the culture surface without being peeled off. In this case, it is possible to estimate a threshold value of the shear stress that causes the cell aggregates 40a to be peeled off, from the boundary position between a region R1 in which the peeling occurs and a region R2 in which the peeling does not occur.

As shown in Fig. 10, the outer shape line of the side surface of the flow channel 55 in planar view may be set linear, and the width w of the flow channel 55 may be linearly changed along the flow direction of the liquid. According to this configuration, the design of the flow channel is facilitated. On the other hand, the magnitude of the change of the shear stress with respect to the position of the liquid in the flow direction is non-linear, and the change in the shear stress with respect to the misregistration of the position of the liquid in the flow direction is large on the flow outlet side (upstream side) of the flow channel 55.

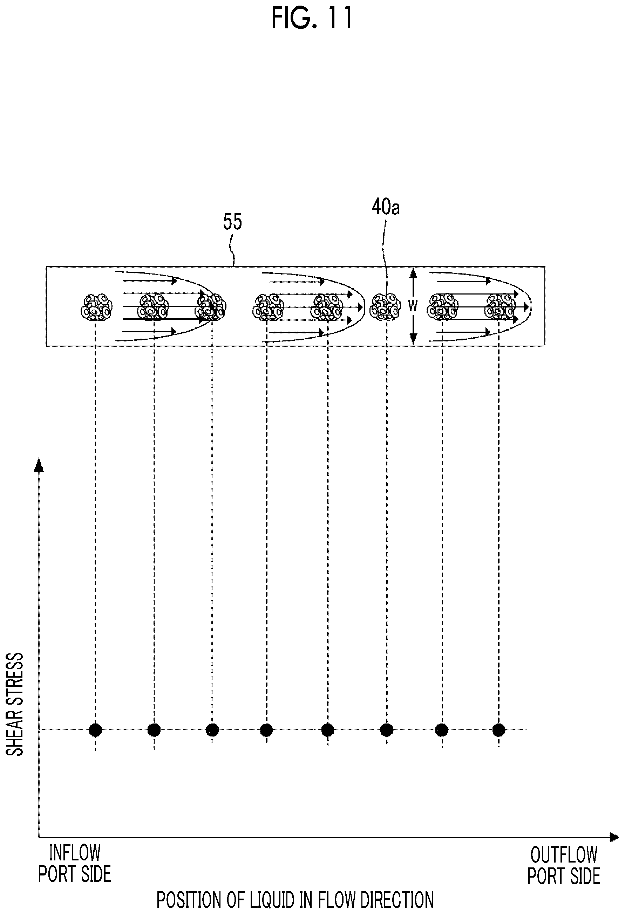

In addition, as shown in Fig. 11, the flow channel cross-sectional area (the width w of the flow channel 55) may be set constant over the entire region in the liquid flow direction. As a result, the shear stress applied to each of the cell aggregates 40a can be made constant at each position in the flow direction of the liquid. According to this configuration, it is possible to evaluate, for example, the variation in the adhesive force of the plurality of cell aggregates 40a.

Fig. 12A and Fig. 12B are each a cross-sectional view showing one example of the configuration of the evaluation device 100 according to the embodiment of the disclosed technology. The evaluation device 100 has a function of supporting the installation of the flow channel member 50 on the culture surface S1. The evaluation device 100 is configured to include the flow channel member 50, an attachment unit 70, a holding part 80, and a fixing unit 90. Hereinafter, the details of each of the above-described constitutional elements of the evaluation device 100 will be described.

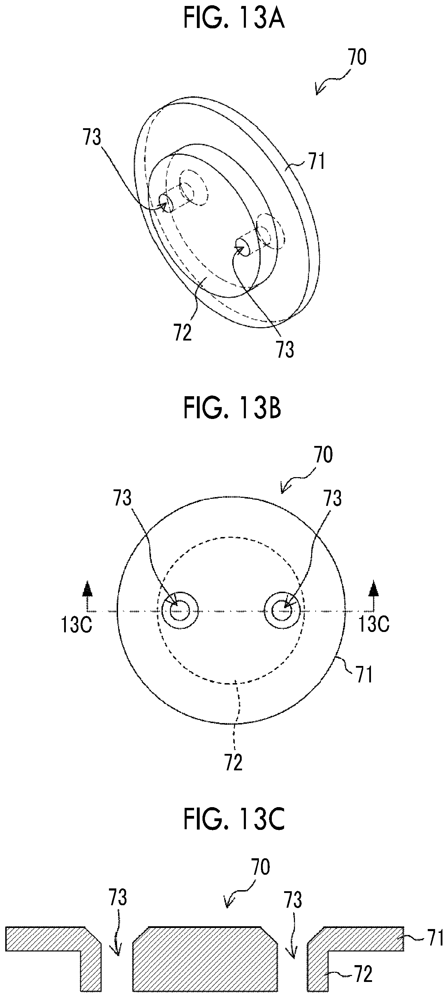

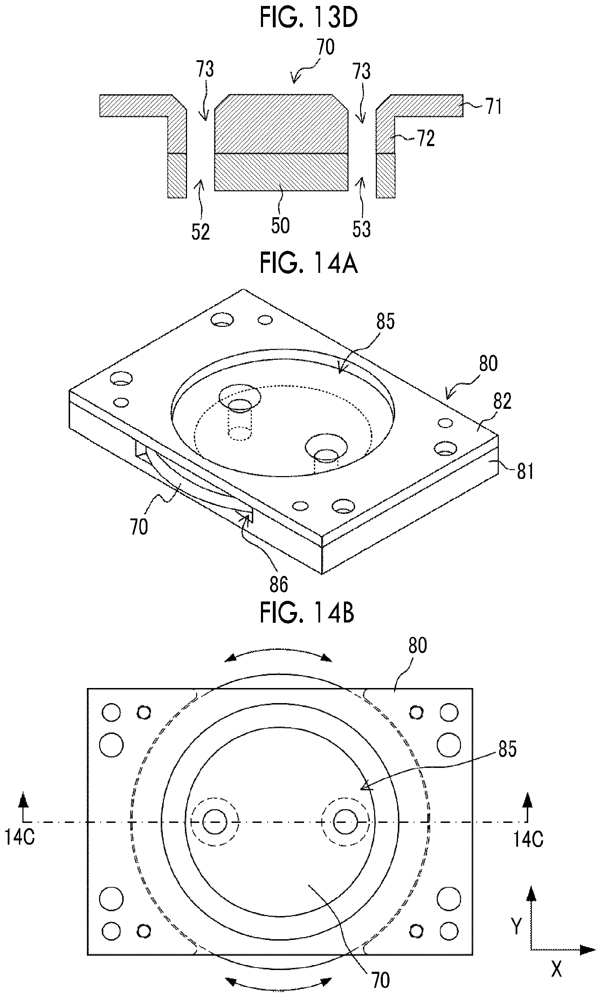

The flow channel member 50 is attached to the attachment unit 70. Fig. 13A and Fig. 13B are respectively a perspective view and a plan view, which show one example of a configuration of the attachment unit 70. Fig. 13C is a cross-sectional view taken along a line 13C-13C in Fig. 13B. Fig. 13D is a cross-sectional view showing a state in which the flow channel member 50 is attached to the attachment unit 70.

The attachment unit 70 has a disk-shaped flange unit 71 and a disk-shaped protruding part 72 having a smaller diameter than the flange unit 71. As shown in Fig. 13D, the flow channel member 50 is attached to a surface of the protruding part 72. The flow channel member 50 is fixed to the attachment unit 70 by the adhesiveness of the flow channel member 50. The flow channel member 50 can be easily detached from the attachment unit 70, and the attachment to and detachment from the attachment unit 70 can be repeated. In a case of being attached with the flow channel member 50, the attachment unit 70 has through-holes 73 that respectively communicate with the inflow port 52 and the outflow port 53 of the flow channel member 50.

It is preferable that the attachment unit 70 is formed from a material having high adhesiveness to the flow channel member 50. In addition, it is preferable that the attachment unit 70 is formed from a material having a light-transmitting property. In a case where the attachment unit 70 is formed from a material having a light-transmitting property, it is easy to carry out registration in a case where the flow channel member 50 is attached to the attachment unit 70. As a material of the attachment unit 70, polycarbonate can be suitably used.

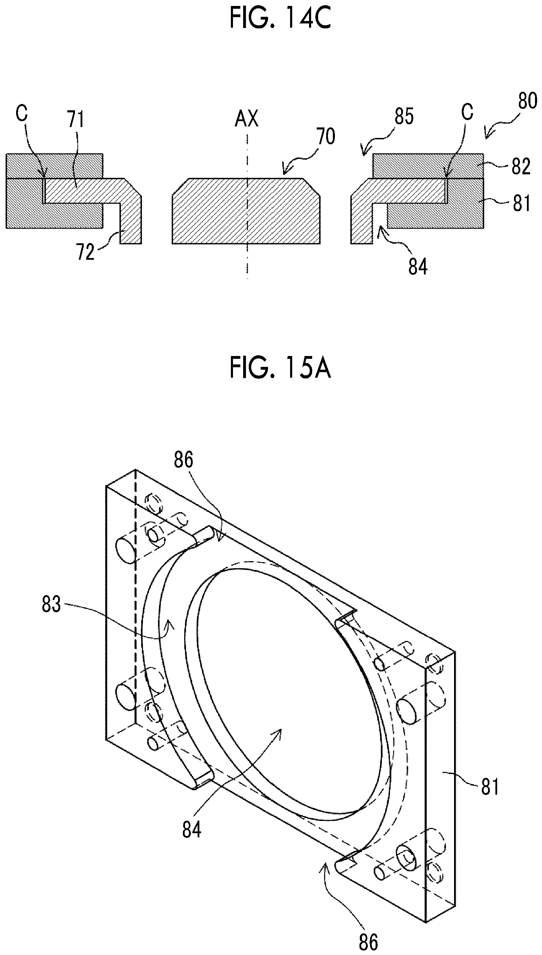

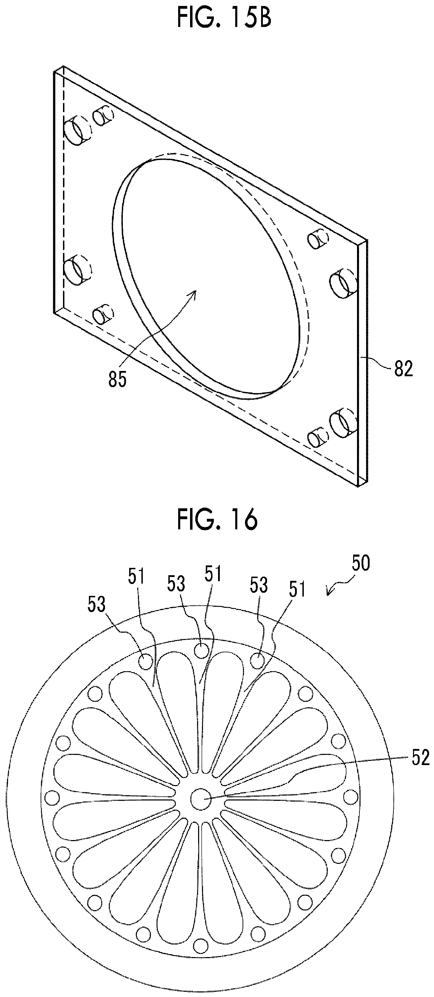

The holding part 80 holds the attachment unit 70 to be rotatable or movable. Fig. 14A and Fig. 14B are respectively a perspective view and a plan view, which show one example of a configuration of the holding part 80 in a state in which the attachment unit 70 according to the embodiment of the disclosed technology is held. Fig. 14C is a cross-sectional view taken along a line 14C-14C in Fig. 14B. Fig. 15A is a perspective view showing one example of a configuration of the base unit 81 constituting the holding part 80, and Fig. 15B is a perspective view showing one example of a configuration of a lid portion 82 constituting the holding part 80.

The base unit 81 has a recessed part 83 into which the flange unit 71 of the attachment unit 70 is fitted, and an opening portion 84 into which the protruding part 72 of the attachment unit 70 is inserted in a state in which the flange unit 71 is fitted into the recessed part 83. In this state, the base unit 81 is covered with the lid portion 82 to sandwich the flange unit 71 between the base unit 81 and the lid portion 82. As a result, the attachment unit 70 is held by the holding part 80 in a state in which the protruding part 72 has protruded from the base unit 81. An opening portion 85 is provided at the center of the lid portion 82, and a surface of the flange unit 71 is exposed in the opening portion 85. A notched part 86 is provided at each of the two end surfaces of the base unit 81 facing each other, and an outer peripheral portion of the flange unit 71 protrudes beyond the notched part 86 to the outside of the holding part 80.

A clearance C of an order of magnitude of tens of micrometers is provided between the side surface of the recessed part 83 provided in the base unit 81 and the flange unit 71, and the attachment unit 70 is rotatable around an axis of a rotation axis AX, in a state in which the attachment unit 70 is held by the holding part 80, and is movable in a plane direction (the XY direction in Fig. 14B) of the culture surface. The rotation and movement of the attachment unit 70 can be carried out by manually operating the outer peripheral portion of the flange unit 71 that has protruded to the outside of the holding part 80. Since the attachment unit 70 is held to be rotatable or movable, it is possible to carry out the registration of the flow channel member 50 with respect to the culture surface in a state in which the flow channel member 50 is attached to the attachment unit 70.

As shown in Fig. 12A and Fig. 12B, the fixing unit 90 has a mounting region R3 in which the substrate 20 having a culture surface is mounted. A groove 91 having a shape corresponding to the outer shape of the substrate 20 is provided in the mounting region R3. In addition, an opening portion 92 is provided in the mounting region R3, and thus it is possible to observe the culture surface S1 through the opening portion 92 from the bottom surface side of the fixing unit 90. The fixing unit 90 fixes the holding part 80 at a position corresponding to the mounting region R3. That is, the relative positional relationship between the mounting region R3 and the holding part 80 is fixed. The holding part 80 may be fastened to the fixing unit 90 using, for example, a bolt 93. The flow channel member 50 is attached to the attachment unit 70, the attachment unit 70 is held by the holding part 80, and the holding part 80 is fastened to the fixing unit 90 in a state in which the substrate 20 is mounted in the mounting region R3 of the fixing unit 90 (see Fig. 12A), whereby the flow channel member 50 is installed on the culture surface S1, and the flow channel 55 is formed on the culture surface S1 (see Fig. 12B).

The fixing unit 90 includes a stopper 94 configured to include a spring, and the holding part 80 is supported by the stopper 94. As a result, in a stage before the holding part 80 is fastened to the fixing unit 90, the flow channel member 50 and the culture surface S1 can be maintained in a non-contact state, and thus in this state, it is possible to carry out the registration of the flow channel member 50 with respect to the culture surface S1.

Hereinafter, a procedure for evaluating cells using the evaluation device 100 will be described. First, the holding part 80 holds the attachment unit 70. Next, the flow channel member 50 is attached to the attachment unit 70. Next, the groove 51, the inflow port 52, the outflow port 53 of the flow channel member 50, and the through-hole 73 of the attachment unit 70 are filled with a liquid. For example, the groove 51, the inflow port 52, the outflow port 53, and the through-hole 73 may be filled with a liquid by carrying out feeding of liquid through a tube that has been inserted into the through-hole 73 in a state in which a surface of the flow channel member 50 on which the groove 51 has been formed is immersed in the liquid. Next, the substrate 20 on which the cells or the cell aggregates 40a are arranged on the culture surface S1 is mounted in the mounting region R3 of the fixing unit 90.

Next, the holding part 80 is attached to the fixing unit 90. Specifically, the bolt 93 for fastening the holding part 80 to the fixing unit 90 is temporarily tightened. In this state, the holding part 80 is supported by the stopper 94, and the flow channel member 50 and the culture surface S1 are maintained in a non-contact state. Next, the holding part 80 is rotated and moved to carry out the registration of the flow channel member 50. The flow channel member 50 is subjected to registration such that the plurality of cell aggregates 40a formed on the culture surface S1 are arranged along the flow direction of the liquid that flows through the flow channel 55. The relative positional relationship between the groove 51 and the cell aggregates 40a can be checked through the substrate 20 that is exposed from the opening portion 92 of the fixing unit 90. The registration of the flow channel member 50 may be carried out using a microscope (not shown in the drawing) installed on the lower side of the fixing unit 90. In addition, the registration of the flow channel member 50 may be carried out using the first alignment mark 201 and the second alignment mark 202 as shown in Fig. 5. After the registration of the flow channel member 50 is completed, the bolt 93 is fully tightened to closely attach the surface of the flow channel member 50 on which the groove 51 has been formed, to the culture surface S1. As a result, the flow channel 55 is formed on the culture surface S1.

Next, the feeding of liquid is carried out through a tube 110 (see Fig. 12B) that has been inserted into the through-hole 73 of the attachment unit 70, and the liquid is allowed to flow through the flow channel 55, whereby a shear stress due to a liquid flow is applied to each of the plurality of cell aggregates 40a. The response of the cell aggregates 40a to the shear stress can be observed through the substrate 20 that is exposed from the opening portion 92 of the fixing unit 90. The observation of the response of the cell aggregate 40a may be carried out using a microscope (not shown in the drawing) installed on the lower side of the fixing unit 90.

As described above, the cell evaluation method according to an embodiment of the disclosed technology includes culturing cells on a culture surface such that a plurality of cells or a plurality of cell aggregates are arranged on a culture surface, installing the flow channel 55 attachable to and detachable from the culture surface, and allowing a liquid to flow through the flow channel 55 to provide a liquid flow to each of the plurality of cells or the plurality of cell aggregates. The flow channel 55 is formed by closely attaching the surface of the flow channel member 50 having the groove 51 on the surface, to the culture surface. The flow channel member 50 is subjected to registration such that the plurality of cells or the plurality of cell aggregates are arranged along a flow direction of the liquid.

Here, in a case of evaluating the response of adhesive cells to the liquid flow, a method of culturing cells or cell aggregates in a closed micro flow channel is considered. In this case, it is necessary to introduce cells into the closed micro flow channel; however, it is not easy to compartmentalize a plurality of cells or a plurality of cell aggregates in the inside of the closed micro flow channel and arrange them along the flow channel. In addition, there is a concern that cells may be damaged in a case where the cells are introduced into the micro flow channel. Further, there is a concern that in the closed micro flow channel, culture medium in a culture medium may be non-uniform, and thus the environment may be different from a standard culture environment. As a result, there is a concern that the state of the cells may be affected, and thus the evaluation result may be affected.

According to the cell evaluation method according to an embodiment of the disclosed technology, since a flow channel that is attachable to and detachable from a culture surface is installed, cells that are cultured in an open system using a commercially available dish can be used as an evaluation target. That is, the culture is possible in a standard environment, and it is also easy to compartmentalize a plurality of cells or a plurality of cell aggregates and arrange them regularly. In addition, it is also easy to quantify or standardize the state of the cells in each compartment. In addition, it is possible to suppress damage to cells during culture, as compared with a case of using a closed micro flow channel. In addition, according to an evaluation method according to an embodiment of the disclosed technology, since it is possible to simultaneously provide a liquid flow to a plurality of cells or a plurality of cell aggregates, which are arranged on a culture surface, it is possible to carry out efficient evaluation. As described above, according to the cell evaluation method according to an embodiment of the disclosed technology, it is possible to simply and efficiently evaluate the response of adhesive cells to a liquid flow.

In addition, the evaluation method according to an embodiment of the disclosed technology includes allowing the plurality of cells or the plurality of cell aggregates to have the same size. As a result, it is possible to quantify or standardize a state of a plurality of cells or a plurality of cell aggregates, which makes it possible to contribute to the acquisition of useful evaluation data.

In addition, the evaluation method according to the embodiment of the disclosed technology may include changing a shear stress in the flow direction of the liquid, where the shear stress due to the liquid flow is applied to each of the plurality of cells or the plurality of cell aggregates. As a result, it is possible to apply shear stresses having magnitudes different from each other, to a plurality of cells or a plurality of cell aggregates, which are arranged on a culture surface, and it is possible to efficiently carry out an evaluation, for example, the estimation of the threshold value of the shear stress that causes the cells or the cell aggregates to be peeled off.

In addition, in the evaluation method according to an embodiment of the disclosed technology, the change in the shear stress along the flow direction of the liquid may be linear. As a result, it is possible to suppress a change in the shear stress with respect to the misregistration in the liquid flow direction of the liquid, as compared with a case where the change in the magnitude of the shear stress along the liquid flow direction is non-linear.

In addition, the evaluation method according to the embodiment of the disclosed technology includes making a shear stress constant at each position in the flow direction of the liquid, where the shear stress due to the liquid flow is applied to each of the plurality of cells or the plurality of cell aggregates. As a result, it is possible to evaluate the variation in the responsiveness to the liquid flow in the plurality of cells or the plurality of cell aggregates.

Fig. 16 is a plan view showing one example of the configuration of the flow channel member 50 according to another embodiment of the disclosed technology. The flow channel member 50 may have a plurality of the grooves 51 that form a flow channel. As a result, it is possible to form a plurality of flow channels on the culture surface, and it is possible to evaluate a large number of cells with one feeding of liquid. In addition, the plurality of grooves 51 may be radially disposed, and the common inflow port 52 may be disposed in the central part.

[Examples]

Hereinafter, the disclosed technology will be described in more detail with reference to Examples. However, the disclosed technology is not limited to the following Examples.

<Patterned culture>

The masking sheet was attached to a surface of a commercially available 35 mm dish (manufactured by Falcon). As the masking sheet, a masking sheet in which 21 opening portions having a square shape and a side length of 200 $\mu$m were arranged on a straight line was used. After bringing the masking sheet into contact with the surface of the dish, the masking sheet was closely attached to the surface of the dish by applying a load to the masking sheet.

Next, a solution of vitronectin of a concentration of 2.5 $\mu$g/ml, which is a cell adhesive, was applied onto the surface of the dish using the masking sheet as a mask, and the dish was allowed to stand for 1 hour. As a result, a pattern of the cell adhesive, corresponding to the pattern of the opening portion of the masking sheet was formed on the surface of the dish. Next, the vitronectin solution was substituted with a phosphate buffer solution, and then the masking sheet was peeled off from the surface of the dish.

Next, a 3% bovine serum albumin solution, which was a blocking agent, was applied onto the surface of the dish. This treatment is a blocking treatment for inhibiting non-specific cell adhesion in a region other than the region in which the cell adhesive has adhered to the culture surface. The treatment time was set to 30 minutes. Thereafter, the blocking agent was substituted with a phosphate buffer solution, and the phosphate buffer solution was aspirated.

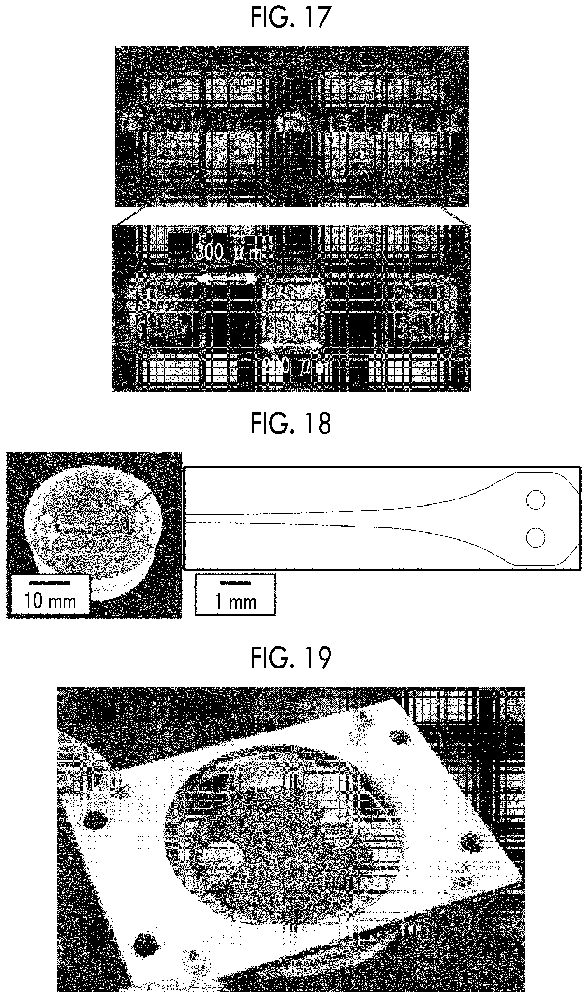

Next, a cell suspension of human-derived iPS cells, which had been adjusted to a concentration of 0.1 M cells/mL, was seeded in a dish, and static culture was carried out for 24 hours. During the culture period, the dish was accommodated in an incubator maintained at 37°C and a $CO_2$ concentration of 5%. Thereafter, the culture medium was replaced to remove floating cells. As a result, a pattern of cell colonies was formed on the surface of the dish. Fig. 17 shows photomicrographic images of the cell colonies patterned by the above-described patterned culture. It was confirmed that a cell pattern corresponding to the pattern of the opening portion of the masking sheet is formed.

installation of flow channel>

A flow channel member was prepared by processing PDMS by a cast molding method. Specifically, PDMS in which a ratio of a main agent to a curing agent was set to 10:1 was poured into a mold and subjected to curing over 3 hours in an environment of 50°C. The PDMS released from the mold was subjected to die cutting with a hollow punch to prepare a flow channel member consisting of PDMS, which had a diameter of 32 mm and a height of 6 mm. The flow channel (groove) was designed such that the shear stress generated in the flow channel due to the liquid flow changed linearly along the flow direction of the liquid. The cross-sectional shape of the flow channel was set to be rectangular, and the height of the flow channel was set to be constant at 200 $\mu$m. The width of the flow channel was derived based on the exact solution obtained by giving a boundary condition of the rectangular cross-sectional flow channel to the Navier-Stokes equation, which is a basic equation of the fluid, and solving the differential equation. The width of the end part of the flow channel on the inflow port side was set to 300 $\mu$m, and the width of the end part thereof on the outflow port side was set to 2,500 $\mu$m. The prepared flow channel member is shown in Fig. 18.

An evaluation device including a flow channel member, an attachment unit, a holding part, and a fixing unit was constructed. The flow channel member was attached to an attachment unit formed from polycarbonate, which has a light-transmitting property. The holding part consisted of a base unit and a lid portion, which consisted of aluminum subjected to an alumite treatment, and the attachment unit was held by sandwiching the attachment unit between the base unit and the lid portion. The attachment unit can be rotated and moved in a plane direction in a state of being held by the holding part. Fig. 19 shows the attachment unit in a state of being held by the holding part.

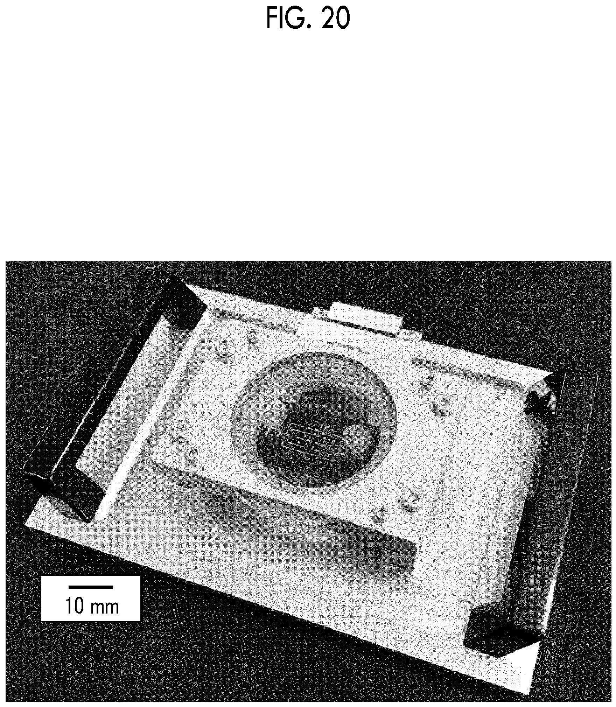

The fixing unit was maintained in an environment of 37°C and a $CO_2$ concentration of 5% and installed in an incubator. The dish in which the cell colonies were arranged was mounted in a mounting region of the fixing unit. The holding part that held the attachment unit to which the flow channel member had been attached was attached to the fixing unit. The registration of the flow channel member was carried out such that the cell colonies on the dish were arranged along the flow channel while observing the positions of the cell pattern and the flow channel (groove formed in the flow channel member) with a microscope. Thereafter, the holding part was fastened to the fixing unit using bolts that were inserted into screw holes provided at the four corners of the holding part, and an evaluation device was constructed. Fig. 20 shows the constructed evaluation device.

<Evaluation of response of adhesive cells to liquid flow>

A culture medium was introduced from the inflow port of the flow channel member using a syringe pump to allow a liquid to flow through the flow channel, whereby a shear stress was applied to the cell colonies arranged on the culture surface. The flow rate of the liquid to be allowed to flow through the flow channel was set to 0.6 ml/min. In this case, a shear stress generated at the end part of the flow channel on the inflow port side is about 5 Pa, and a shear stress generated at the end part of the flow channel on the outflow port side is about 0.5 Pa.

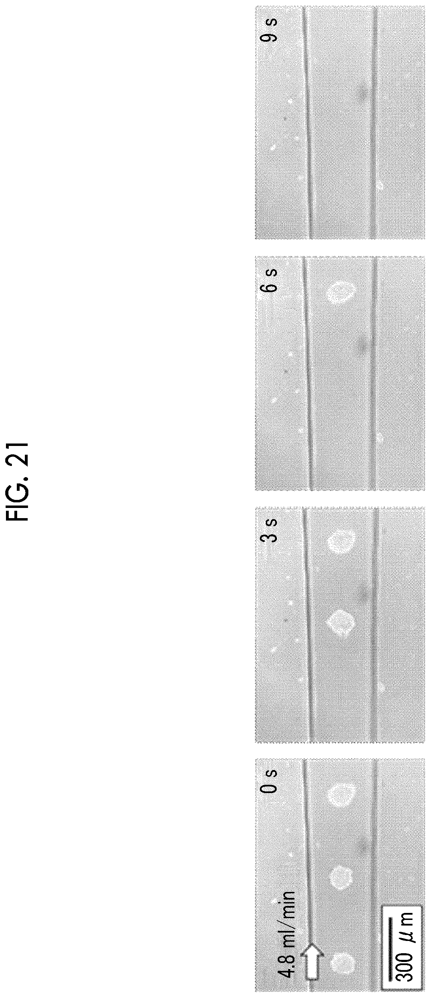

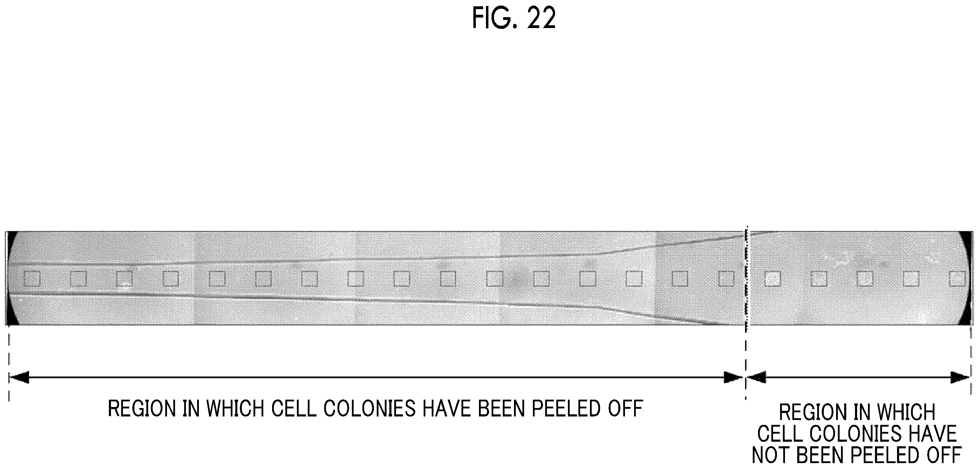

Fig. 21 shows photomicrographic images showing a state in which cell colonies arranged on the culture surface are peeled off by a shear stress due to a liquid flow. As shown in Fig. 21, a state, in which the cell colonies were peeled off in order from the upstream side of the flow channel, where the shear stress was relatively large, was observed. Fig. 22 shows photomicrographic images showing a state of the entire flow channel after providing a liquid flow. As shown in Fig. 22, a plurality of cell colonies positioned on the downstream side of the flow channel, where the shear stress was relatively small, were not peeled off but maintained in a state of being adhered to the culture surface. It was estimated that the threshold value of the shear stress that causes the cell colonies to be peeled off from the boundary position between the region in which the cell colonies have been peeled off and the region in which the cell colonies have not been peeled off is 1.2 Pa.

As described above, it was confirmed that it is possible to simply and efficiently evaluate the response of adhesive cells to a liquid flow and it is also possible to realize the quantification of the responsiveness of the cells.

It is noted that the disclosure of JP2022-020642 filed on February 14, 2022, is incorporated in the present specification in its entirety by reference. In addition, all documents, patent applications, and technical standards described in the present specification are incorporated in the present specification by reference, to the same extent as in the case where each of the documents, patent applications, and technical standards is specifically and individually described.

**Claims**

**1.** A cell evaluation method, comprising:

culturing cells such that a plurality of cells or a plurality of cell aggregates are arranged on a culture surface;
installing a flow channel attachable to and detachable from the culture surface; and
allowing a liquid to flow through the flow channel to provide a liquid flow to each of the plurality of cells or the plurality of cell aggregates.

2. The evaluation method according to claim 1,
wherein a surface of a flow channel member having, on the surface, a groove constituting the flow channel is closely attached to the culture surface to form the flow channel.

3. The evaluation method according to claim 2,
wherein the flow channel member is subjected to registration such that the plurality of cells or the plurality of cell aggregates are arranged along a flow direction of the liquid.

4. The evaluation method according to claim 3,
wherein the registration of the flow channel member is carried out using a first alignment mark provided on a surface of the flow channel member and a second alignment mark provided on the culture surface.

5. The evaluation method according to any one of claims 1 to 4,
wherein a shear stress applied to each of the plurality of cells or the plurality of cell aggregates due to the liquid flow changes along a flow direction of the liquid.

6. The evaluation method according to claim 5,
wherein a shear stress applied to each of the plurality of cells or the plurality of cell aggregates due to the liquid flow changes linearly along a flow direction of the liquid.

7. The evaluation method according to claim 5 or 6,
wherein an area of a cross section of the flow channel intersecting with the flow direction of the liquid changes along the flow direction of the liquid.

8. The evaluation method according to claim 7,
wherein an area of a cross section of the flow channel intersecting with the flow direction of the liquid expands from an upstream side toward a downstream side in the flow direction of the liquid.

9. The evaluation method according to any one of claims 1 to 4,
wherein a shear stress applied to each of the plurality of cells or the plurality of cell aggregates due to the liquid flow is constant at each position in the flow direction of the liquid.

10. The evaluation method according to any one of claims 1 to 9,
wherein the culturing of cells is carried out such that the plurality of cells or the plurality of cell aggregates form a predetermined pattern on the culture surface.

11. The evaluation method according to any one of claims 1 to 10,

wherein a sheet having a plurality of opening portions is attached to the culture surface, and
the plurality of cells or the plurality of cell aggregates are formed in a portion of the culture surface, which is exposed in each of the opening portions.

12. The evaluation method according to any one of claims 1 to 11,
wherein the plurality of cells or the plurality of cell aggregates have the same size.

13. An evaluation device for providing a liquid flow to a plurality of cells or a plurality of cell aggregates, which are arranged on a culture surface,

wherein a flow channel attachable to and detachable from the culture surface is formed, and
a liquid is allowed to flow through the flow channel to provide a liquid flow to each of the plurality of cells or the plurality of cell aggregates.

14. The evaluation device according to claim 13, comprising:

a flow channel member having, on a surface, a groove constituting the flow channel,
wherein the surface of the flow channel member which has the groove is closely attached to the culture surface to form the flow channel.

**15.** The evaluation device according to claim 14,
wherein the flow channel is configured such that a shear stress applied to each of the plurality of cells or the plurality of cell aggregates changes along a flow direction of the liquid.

**16.** The evaluation device according to claim 15,
wherein the flow channel is configured such that a shear stress applied to each of the plurality of cells or the plurality of cell aggregates changes linearly along a flow direction of the liquid.

**17.** The evaluation device according to claim 15 or 16,
wherein an area of a cross section of the flow channel intersecting with the flow direction of the liquid changes along the flow direction of the liquid.

**18.** The evaluation device according to claim 17,
wherein an area of a cross section of the flow channel intersecting with the flow direction of the liquid expands from an upstream side toward a downstream side in the flow direction of the liquid.

**19.** The evaluation device according to any one of claims 14 to 18, further comprising:

an attachment unit to which the flow channel member is attached;
a holding part that holds the attachment unit to be rotatable or movable; and
a fixing unit that has a mounting region, in which a substrate having the culture surface is mounted, and fixes the holding part to a position corresponding to the mounting region.

**20.** The evaluation device according to claim 19,
wherein the flow channel member and the attachment unit are formed from a material having a light-transmitting property.

## FIG. 1

## FIG. 2A

## FIG. 2B

## FIG. 2C

## FIG. 2D

## FIG. 2E

## FIG. 2F

## FIG. 2G

# FIG. 3A

# FIG. 3B

## FIG. 4A

## FIG. 4B

## FIG. 5

# FIG. 6

55

w

40a

# FIG. 7

SHEAR STRESS

INFLOW
PORT SIDE

OUTFLOW
PORT SIDE

POSITION OF LIQUID IN FLOW DIRECTION

# FIG. 8

$y$

$h/2$

$-w/2$ $w/2$ $z$

$-h/2$

NONDIMENSIONALIZATION

$y$

$1$

$-\gamma$ $\gamma$ $z$

$-1$

$$\gamma = \frac{w}{h}$$

## FIG. 9

40a

55

R1

R2

## FIG. 10

55

40a

W

SHEAR STRESS

INFLOW
PORT SIDE

OUTFLOW
PORT SIDE

POSITION OF LIQUID IN FLOW DIRECTION

# FIG. 11

55   40a

W

SHEAR STRESS

INFLOW
PORT SIDE

OUTFLOW
PORT SIDE

POSITION OF LIQUID IN FLOW DIRECTION

## FIG. 12A

## FIG. 12B

## FIG. 13A

70

73

71

72

73

## FIG. 13B

70

73          73

13C          13C

71

72

## FIG. 13C

70

73          73

71

72

## FIG. 13D

## FIG. 14A

## FIG. 14B

# FIG. 14C

# FIG. 15A

## FIG. 15B

85    82

## FIG. 16

50

53  51  53  51  53  51

52

## FIG. 17

## FIG. 18

## FIG. 19

FIG. 20

10 mm

FIG. 21

FIG. 22

REGION IN WHICH CELL COLONIES HAVE BEEN PEELED OFF

REGION IN WHICH
CELL COLONIES HAVE
NOT BEEN PEELED OFF

EP 4 461 815 A1

## INTERNATIONAL SEARCH REPORT

| International application No. |
|---|
| **PCT/JP2022/044238** |

**A. CLASSIFICATION OF SUBJECT MATTER**

*C12Q 1/02*(2006.01)i; *C12M 3/00*(2006.01)i
FI: C12Q1/02; C12M3/00

According to International Patent Classification (IPC) or to both national classification and IPC

**B. FIELDS SEARCHED**

Minimum documentation searched (classification system followed by classification symbols)

C12Q1/00-3/00; C12M1/00-3/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2021-104015 A (UNIV TOKYO) 26 July 2021 (2021-07-26)<br>claims, paragraphs [0021]-[0030], [0054]-[0057], examples, fig. 2, 4-5 | 1-10, 12-20 |
| Y | | 11 |
| Y | WO 2021/256452 A1 (SHINETSU POLYMER CO) 23 December 2021 (2021-12-23)<br>claims, paragraphs [0002]-[0005] | 11 |
| A | JP 2012-100642 A (TOKYO WOMEN'S MEDICAL COLLEGE) 31 May 2012 (2012-05-31)<br>claims, examples | 1-20 |
| A | WO 2020/195458 A1 (FUJIFILM CORPORATION) 01 October 2020 (2020-10-01)<br>claims | 1-20 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **15 December 2022** | **14 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

33

INTERNATIONAL SEARCH REPORT
Information on patent family members

International application No.

**PCT/JP2022/044238**

| Patent document cited in search report | | | Publication date (day/month/year) | Patent family member(s) | | | Publication date (day/month/year) |
|---|---|---|---|---|---|---|---|
| JP | 2021-104015 | A | 26 July 2021 | (Family: none) | | | |
| WO | 2021/256452 | A1 | 23 December 2021 | (Family: none) | | | |
| JP | 2012-100642 | A | 31 May 2012 | (Family: none) | | | |
| WO | 2020/195458 | A1 | 01 October 2020 | US<br>claims<br>EP | 2022/0082503<br>3936603 | A1<br>A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012100642 A
- WO 2015125742 A
- JP 2022020642 A

**Non-patent literature cited in the description**

- **T. OHASHI et al.** *JSME Inter. J. Series C*, 2000, vol. 43 (4), 780-786